# EUROPÄISCHE PATENTSCHRIFT

(11) **EP 1 514 864 B1**
(45) Veröffentlichungstag und Bekanntmachung des Hinweises auf die Patenterteilung: **19.03.2008**
(21) Anmeldenummer: 04020499.2
(22) Anmeldetag: 28.08.2004
(51) Int. Cl.: C07C 211/29, C07C 255/50, C07C 33/46

(54) **Difluoralkylaromaten**
Difluoralkyl aromatic compounds
Composés aromatiques difluoro alcoylés

(30) Priorität: 09.09.2003 DE 10341534
(43) Veröffentlichungstag der Anmeldung: 16.03.2005
(73) Patentinhaber: Saltigo GmbH, 40764 Langenfeld (DE)
(72) Erfinder: Hilgers, Petra, Dr., 51503 Rösrath (DE); Marhold, Albrecht, Dr., 51373 Leverkusen (DE)
(74) Vertreter: Wichmann, Birgid

(56) Entgegenhaltungen:
- EP-A- 0 245 690
- US-A- 5 547 975
- QIU, JIAN ET AL: "2,6-Difluorophenol as a Bioisostere of a Carboxylic Acid: Bioisosteric Analogs of .gamma.-Aminobutyric Acid" JOURNAL OF MEDICINAL CHEMISTRY , 42(2), 329-332 CODEN: JMCMAR; ISSN: 0022-2623, 1999, XP002305237

## Beschreibung

Die vorliegende Erfindung betrifft 3,4-Difluor-2-alkylaromaten und 2,4-Difluor-3-alkylaromaten, ein Verfahren zu deren Herstellung sowie ihre Verwendung zur Herstellung von Wirkstoffen.

2,3,4-Trisubstituierte Aromaten wie insbesondere solche die Fluor oder fluorhaltige Gruppen als Substituenten tragen sind wichtige Bausteine für Wirkstoffe in Arzneimitteln, wie vor allem in analgetisch und antibakteriellen Wirkstoffen und Pflanzenschutzmitteln, wie vor allem in Herbiziden und Fungiziden (siehe auch EP-A 864 559, EP-A 609 798, WO 95/31446, EP-A 625 505, DE-A 36 157 67 und WO 98/46608).

Es ist deshalb wünschenswert, 2,3,4-trisubstituierte Aromaten bereitzustellen, mit denen die Lipophilie des gesamten Wirkstoffmoleküls und damit dessen Wirksamkeit positiv beeinflusst werden kann und die sich als Synthesebaustein besonders breit verwenden lassen.

Weiterhin ist es wünschenswert, ein Verfahren bereitzustellen, mit dem diese 2,3,4-trisubstituierten Aromaten in guten Ausbeuten ausgehend von einfach erhältlichen Edukten gewonnen werden können.

Beispielsweise ist 2,4-Difluor-3-methylbenzoesäure aus WO 99/14214 und WO 99/35117 bekannt. Die Herstellung umfasst dabei zunächst die Umsetzung von 2,4-Difluorbrombenzol mit einer starken Base und einem Methylierungsreagenz zu 2,6-Difluor-3-bromtoluol sowie weiterhin die Überführung in ein metallorganisches Reagenz und anschließende Behandlung mit Kohlendioxid.

Gemäß DE-A 36 157 67 wird 2,4-Difluor-3-methylbenzoesäure erhalten, indem 2,4-Difluor-3-methylacetophenon mit Brom und Natronlauge oxidiert wird.

Die Herstellung von 3,4-Difluor-2-methyl-benzoesäure ist beispielsweise aus EP-A 609 798, WO 95/31446 und EP-A 625 505 bekannt und erfolgt durch Dilithiierung von 3,4-Difluorbenzoesäure und anschließende Umsetzung mit Methyliodid.

Nachteilig an den genannten Verfahren ist die Tatsache, dass der mehrfache Einsatz metallorganischer Reagentien im industriellen Maßstab aus sicherheitstechnischen Gründen kritisch und insgesamt sehr teuer ist und die genannten Verfahren für jedes Produkt unterschiedlich sind.

Es wurden nun Verbindungen der Formel (I) gefunden 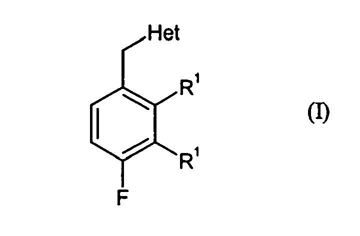 in der
Het für Amino oder Hydroxy steht und einer der Reste R¹ für Fluor und der andere Rest R¹ für C₁-C₄-Alkyl steht.

Bevorzugte Verbindungen der Formel (I) sind:

3,4-Difluor-2-methyl-benzylalkohol, 2,4-Difluor-3-methyl-benzylalkohol, 3,4-Difluor-2-methyl-benzylamin und 2,4-Difluor-3-methyl-benzylamin.

Vorzugsweise stellt man die Verbindungen der Formel (I) so her, dass
- in einem Schritt A)
   Verbindungen der Formel (II) 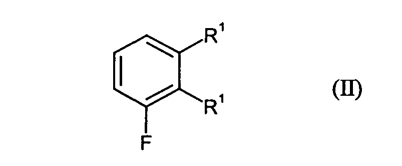 in Gegenwart von Brom und einem Katalysator zu Verbindungen der Formel (III) umgesetzt werden 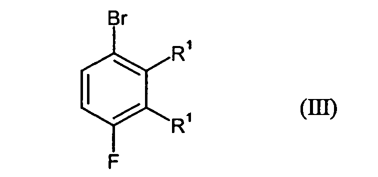 und für den Fall, dass Het für Amino steht, die Verbindungen der Formel (III)
- in einem Schritt B1 mit Cyanid zunächst zu Verbindungen der Formel (IV) 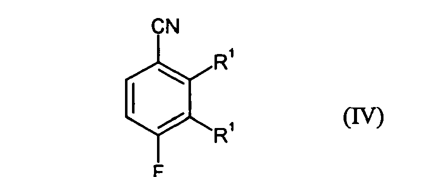 und anschließend
- in einem Schritt B2 durch Reduktion in die entsprechenden Verbindungen der Formel (I) überführt werden oder
   für den Fall, dass Het für Hydroxy steht,
- in einem Schritt B3 die Verbindungen der Formel (III) durch Überführung in eine magnesiumorganische Verbindung, Umsetzung dieser magnesiumorganischen Verbindung mit Kohlendioxid und anschließender Behandlung mit Säure zu Verbindungen der Formel (V) umsetzt 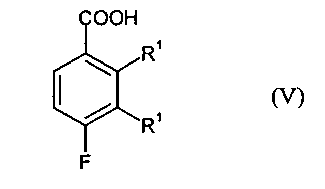 und die Verbindungen der Formel (V)
- in einem Schritt B4 durch Umsetzung mit einem Halogenierungsmittel in Verbindungen der Formel (VI) überführt werden 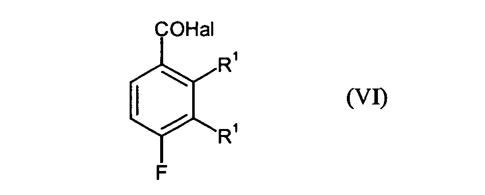 in der Hal für Brom oder Chlor steht
- und in einem Schritt B5 die Verbindungen der Formel (VI) durch Reduktion in die entsprechenden Verbindungen der Formel (I) überführt werden.

Alternativ zu den Schritten B3 bis B5 ist auch
- in einem Schritt B6 die Umsetzung der Verbindungen der Formel (III) durch Überführung in eine magnesiumorganische Verbindung, Umsetzung dieser magnesiumorganischen Verbindung mit einem N,N-substituierten-Ameisensäureamid und anschließender Behandlung mit Säure zu Verbindungen der Formel (VII) möglich 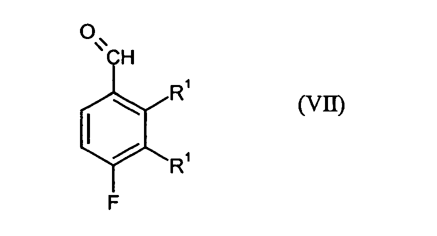
wobei dann
- in einem Schritt B7 die Verbindungen der Formel (VII) durch Reduktion in die entsprechenden Verbindungen der Formel (I) überführt werden.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

**C₁-C₄-Alkyl** bedeutet jeweils unabhängig einen geradkettigen, verzweigten oder unverzweigten Alkyl-Rest, wie vorzugsweise Methyl, Ethyl, n-Propyl, iso-Propyl, n-Butyl und tert.-Butyl.

Von der Erfindung sind als unverzichtbare Intermediate mit umfasst:
Verbindungen der Formel (III) mit Ausnahme von 3-Brom-2,6-difluortoluol.
Verbindungen der Formel (IV).
Verbindungen der Formel (V) mit Ausnahme von 2,4-Difluor-3-methylbenzoesäure und 3,4-Difluor-2-methylbenzoesäure.
Verbindungen der Formel (VI) mit Ausnahme von 2,4-Difluor-3-methylbenzoesäure-chlorid.
Verbindungen der Formel (VII).

Bevorzugte Verbindung der Formel (III) ist 2-Brom-5,6-difluortoluol.

Bevorzugte Verbindungen der Formel (IV) sind 3,4-Difluor-2-methylbenzoesäurenitril und 2,4-Difluor-3-methylbenzoesäurenitril.

Bevorzugte Verbindung der Formel (VI) ist 3,4-Difluor-2-methylbenzoesäurechlorid.

Bevorzugte Verbindungen der Formel (VII) sind 3,4-Difluor-2-methylbenzaldehyd und 2,4-Difluor-3-methylbenzaldehyd.

Die Bromierung gemäß Schritt A) erfolgt mit Brom und in Gegenwart von Katalysator, wobei als Katalysator vorzugsweise Eisen und Eisensulfid eingesetzt werden. Die Reaktion kann beispielsweise in einem Temperaturbereich von -10 bis 50°C, vorzugsweise bei 0 bis 30°C durchgeführt werden.

Die Umsetzung gemäß Schritt B1 mit Cyanid wird vorzugsweise so durchgeführt, dass die Verbindungen der Formel (III) mit einem Metallcyanid, vorzugsweise Alkalimetall- und/oder Kupfercyanid, in einem aprotisch-polaren Lösungsmittel in einem Temperaturbereich von beispielsweise 50 bis 200°C, vorzugsweise von 130 bis 170°C umgesetzt werden.

Aprotisch-polare Lösungsmittel sind beispielsweise Nitrile, wie Acetonitril, Propionitril oder Benzonitril; Amide, wie N,N-Dimethylformamid, N,N-Dimethylacetamid, N-Methylformanilid, N-Methylpyrrolidon oder N,N-Dimethylimidazolin-2-on, Sulfoxide wie Dimethylsulfoxid, Sulfone wie Tetramethylensulfon oder Mischungen der genannten Lösungsmittel.

Die Reduktion gemäß Schritt B2 kann vorzugsweise durch Umsetzung der Verbindungen der Formel (IV) mit komplexen Aluminiumwasserstoffverbindungen in einem inerten Lösungsmittel bei einer Reaktionstemperatur von beispielsweise -30 bis 70°C, bevorzugt zwischen 0 bis 30°C erfolgen.

Komplexen Aluminiumwasserstoffverbindungen sind beispielsweise solche der Formel (VIII)

Met[AlH_{q}R² _{(4-q)}]ₚ (VIII)

in der
- Met: für ein ein- oder zweiwertiges Metall wie vorzugsweise Zink, Lithium, Natrium oder Kalium steht und
- R²: für C₁-C₈-Alkyl steht und
- q: für 1, 2, 3 oder 4, bevorzugt für 4 oder 1 und
- p: für die Wertigkeit von Met steht.

### Besonders bevorzugt ist Lithiumaluminiumhydrid

Inerte Lösungsmittel sind beispielsweise aromatische oder aliphatische Kohlenwasserstoffe wie Toluol, Xylol, verschiedene Petrolether, Hexan, Cyclohexan und Ether, wie Diethylether, Methyl-tert.-butylether, Diisopropylether, Dioxan, Tetrahydrofuran oder Ethylenglykoldimethyl- oder -diethylether oder Gemische solcher organischen Lösungsmittel.

Alternativ ist die katalytische Hydrierung in einem protischen Lösungsmittelmöglich, wobei sich als Katalysatoren insbesondere solche eignen, die Palladium, Rhodium, Iridium oder Platin enthalten, wobei Palladium bevorzugt ist. Besonders bevorzugt als Katalysator ist Palladium auf Kohle mit einem Gehalt von 0,1 bis 20 Gew.-%.

Protische Lösungsmittel sind beispielsweise Alkohole, wie Methanol, Ethanol, n- oder i-Propanol, Ethylenglykolmonomethylether, Ethylenglykolmonoethylether, Diethylenglykolmonomethylether, Diethylenglykolmonoethylether oder Gemische davon.

Die Umsetzung gemäß Schritt B3 kann vorzugsweise durch Umsetzung der Verbindungen der Formel (III) mit Magnesium oder Grignardreagentien wie beispielsweise Isopropylmagnesiumbromid in einem inerten Lösungsmittel gemäß obiger Definition in einem Temperaturbereich von -20 bis 70°C, vorzugsweise bei 0 bis 30°C erfolgen.

Die anschließende Reaktion mit Kohlendioxid kann durch Einleiten von gasförmigem oder durch Umsetzung mit festem Kohlendioxid erfolgen. Die anschließende saure Aufarbeitung kann beispielsweise in an sich bekannter Weise durch Ansäuren mit einer wässrigen Säure und Extraktion mit einem organischen Lösungsmittel erfolgen.

Analog dazu kann die Reaktion gemäß B6 durchgeführt werden. In diesem Fall ist ein bevorzugtes N,N-substituiertes Ameisensäureamid N,N-Dimethylformamid.

Das entsprechende Benzoesäurechlorid wird dargestellt, indem man die Benzoesäure mit einem Chlorierungsmittel, wie z.B. Thionylchlorid, in einem unpolar-aprotsichen Lösungsmittel, wie z.B. Dichlormethan, und einer katalytischen Menge Dimethylformamid in einem Temperaturbereich von 10 - 50°C, vorzugsweise bei 20 - 25°C, zur Reaktion bringt.

Die Umsetzung zu Verbindungen der Formel (VI) gemäß Schritt B4 kann vorzugsweise durch Umsetzung der Verbindungen der Formel (V) mit Thionylchlorid, Thionylbromid, Phosphoroxychlorid, Phosphorylchlorid oder Phosgen erfolgen.

Die Reduktion gemäß Schritt B5 kann vorzugsweise durch Umsetzung der Verbindungen der Formel (VI) mit komplexen Elementwasserstoffverbindungen in einem inerten Lösungsmittel gemäß obiger Definition bei einer Reaktionstemperatur von beispielsweise -30 bis 70°C, bevorzugt zwischen 0 bis 30°C erfolgen.

Komplexe Elementwasserstoffverbindungen sind beispielsweise solche der Formel (IX)

Met[EH_{q}R³ _{(4-q)}]ₚ (IX)

in der
- Met: für ein ein- oder zweiwertiges Metall wie vorzugsweise Zink, Lithium, Natrium oder Kalium steht und
- E: für Aluminium oder Bor
- R³: für C₁-C₈-Alkyl steht und
- q: für 1, 2, 3 oder 4, bevorzugt für 4 oder 1 und
- p: für die Wertigkeit von Met steht.

### Besonders bevorzugt ist Natriumborhydrid

Analog dazu kann die Reduktion gemäß Schritt B7 durchgeführt werden.

Auf erfindungsgemäße Weise erhält man die Verbindungen der Formel (I) in guten Ausbeuten.

Die erfindungsgemäßen Verbindungen der Formel (I) eignen sich insbesondere als Bausteine für Wirkstoffe in Arzneimitteln, wie vor allem in analgetisch und antibakteriellen Wirkstoffen und Pflanzenschutzmitteln, wie vor allem in Herbiziden und Fungiziden oder Zwischenprodukten davon.

### Beispiele:

### Beispiel 1

### Herstellung von 2-Brom-5,6-difluortoluol

300 g (2.34 mol) 2,3-Difluortoluol und 7.9 g (141 mmol) Eisen wurden vorgelegt und 374 g (2.34 mol) Brom innerhalb von 4 Stunden langsam zugetropft, wobei die Temperatur durch Kühlung unter 30°C gehalten wurde. Man ließ über Nacht (8 Stunden) bei Raumtemperatur nachrühren. Das Reaktionsgemisch wurde mit 300 ml Wasser und 300 ml Methyl-tert.butylether verdünnt. Die Phasen wurden getrennt und die wässrige Phase gründlich mit Methyl-tert.butylether extrahiert. Die vereinigten organischen Phasen wurden mit Natriumthiosulfatlösung und Wasser gewaschen, getrocknet und eingeengt. Destillation über eine Vigreux-Kolonne lieferte 293 g farblose Flüssigkeit; Sdp. = 68-70°C bei 57 mbar.

### Beispiel 2

### Herstellung von 3,4-Difluor-2-methylbenzoesäure

Zu einer Lösung von 35 g (170 mmol) 2-Brom-5,6-difluortoluol aus Beispiel 1 in 80 ml THF wurden unter Stickstoff und Kühlung 26.2 g (255 mmol) einer 2 M Lösung von 2-Propylmagnesiumchlorid in THF zugetropft. Nach vollständiger Zugabe wurde 15 Stunden bei Raumtemperatur nachgerührt. Dann wurde langsam über 5 Stunden Kohlendioxid eingeleitet und 1 Stunde nachgerührt. Zur Aufarbeitung wurde langsam Wasser zugetropft, und die Lösung mit konzentrierter Salzsäure auf pH 1 angesäuert. Die wässrige Phase wurde mehrfach mit Methyltert.butylether und Essigsäureethylester extrahiert. Die vereinigten organischen Phasen wurden mit gesättigter Natriumchloridlösung gewaschen, mit Magnesiumsulfat getrocknet und eingeengt. Der feste Rückstand wurde aus Dichlormethan umkristallisiert.

Man erhielt 21 g farblosen Feststoff; Schmp.: 135°C.

### Beispiel 3

### Herstellung von 3,4-Difluor-2-methylbenzoesäurechlorid

32.0 g (185 mmol) 3,4-Difluor-2-methylbenzoesäure aus Beispiel 2 wurden unter Stickstoff in 350 ml Dichlormethan vorgelegt und 36.8 g (309 mmol) Thionylchlorid innerhalb von 5 Minuten zugetropft. Man erhitzte 27 Stunden auf Rückfluss und destillierte dann Thionylchlorid und Dichlormethan ab. Destillation des Rückstands lieferte 30 g farblose Flüssigkeit; Sdp.: 35°C bei 2.2 mbar.

### Beispiel 4

### Herstellung von 3,4-Difluor-2-methylbenzoesäurenitril

54 g (261 mmol) 2-Brom-5,6-difluortoluol aus Beispiel 1, 28 g (313 mmol) Kupfercyanid und 9.9 g (52 mmol) Kupferiodid wurden unter Stickstoff in 600 ml Dimethylformamid vorgelegt. Man erhitzte 16 Stunden lang unter Rückfluss. Der entstandene Feststoff wurde abfiltriert und mehrfach mit wenig Methyl-tert.-butylether gewaschen. Das Filtrat wurde mit 400 ml Wasser versetzt und die organischen Phase abgetrennt. Die wässrige Phase wurde viermal mit je ca. 200 ml Methyl-tert.-butylether extrahiert. Die vereinigten organischen Phasen wurden mit Wasser gewaschen, mit Magnesiumsulfat getrocknet und eingeengt. Man erhielt 35.5 g eines gelben Feststoffs; Schmp.: 37 - 38.5°C.

### Beispiel 5

### Herstellung von 3,4-Difluor-2-methylbenzaldehyd

Unter Stickstoff wurden 1.3 g (53 mmol) Magnesium in 65 ml Tetrahydrofuran vorgelegt und über Nacht mit einem Magnetrührer langsam gerührt. Der Rührer wurde abgestellt und ein Iod-Kristall zur Lösung gegeben. Dann tropfte man 10 g (48 mmol) 2-Brom-5,6-difluortoluol aus Beispiel 2 so langsam zu, dass ein kontinuierlicher leichter Rückfluss erhalten wurde. Nach beendeter Zugabe wurde nachgerührt, bis eine klare Lösung entstand (ca. 2 Stunden). Dann tropft man innerhalb von 100 Minuten bei Raumtemperatur 7.1 g (97 mmol) Dimethylformamid in 35 ml Tetrahydrofuran zu. Die Reaktionsmischung wurde 4 Stunden unter Rückfluss erhitzt, dann durch Gießen auf Eiswasser hydrolysiert. Die restlichen Magnesium-Späne wurden abfiltriert. Man stellte die Suspension mit konzentrierte Salzsäure sauer (pH 1). Die wässrige Phase extrahierte man 3 mal mit jeweils 50 ml Ethylacetat. Die vereinigten organischen Phasen wurden mit 100 ml gesättigter Natriumchloridlösung gewaschen, getrocknet und eingeengt.

Man erhielt 13 g eines farblosen Öls; Sdp.: 40°C (0.9 mbar).

### Beispiel 6

### Herstellung von 3,4-Difluor-2-methylbenzylamin

33 g (213 mmol) 3,4-Difluor-2-methylbenzoesäurenitril aus Beispiel 5 wurden unter Stickstoff in 300 ml Tetrahydrofuran aufgenommen. Zu dieser Lösung wurden 8.1 g (213 mmol) Lithiumaluminiumhydrid portionsweise zugegeben und anschließend 6 Stunden bei Raumtemperatur nachgerührt. Man hydrolysierte überschüssiges Lithiumaluminiumhydrid durch vorsichtige Zugabe von Wasser und filtrierte den entstandenen Niederschlag ab. Das erhaltene Filtrat wurde mit Wasser gewaschen und die vereinigten wässrigen Phasen mit Methyl-tert.butylether extrahiert. Die vereinigten organischen Phasen wurden mit gesättigter Natriumchloridlösung gewaschen, mit Magnesiumsulfat getrocknet und eingeengt.

Destillation lieferte 17 g dunkelrotes Harz; Sdp.: 65 - 70 °C bei 2.3 -1.8 mbar.

### Beispiel 7

### Herstellung von 3,4-Difluor-2-methylbenzylalkohol

12.6 g (66 mmol) 3,4-Difluor-2-methylbenzoesäurechlorid aus Beispiel 3 wurden unter Stickstoff in einem Gemisch aus 40 ml Dimethylformamid und 40 ml Tetrahydrofuran vorgelegt. Man gab portionsweise 5 g (132 mmol) Natriumborhydrid zu und ließ anschließend noch 2 Stunden bei Raumtemperatur rühren. Das Reaktionsgemisch wurde unter Eiskühlung mit 100 ml Eiswasser versetzt und dreimal mit jeweils 70 ml Dichlormethan extrahiert. Die vereinten organischen Phasen wurden über Magnesiumsulfat getrocknet und eingeengt. Destillation lieferte 9 g farblose Flüssigkeit; Sdp.: 27°C bei 1.4 mbar.

### Beispiel 8

### Herstellung von 3-Brom-2,6-difluortoluol

300 g (2.34 mol) 2,6-Difluortoluol und 7.9 g (141 mmol) Eisen wurden vorgelegt und 374 g (2.34 mol) Brom innerhalb von 4 Stunden langsam zugetropft, wobei die Temperatur durch Kühlung unter 30°C gehalten wurde. Man ließ über Nacht (8 Stunden) bei Raumtemperatur nachrühren. Das Reaktionsgemisch wurde mit 300 ml Wasser und 300 ml Dichlormethan verdünnt. Die Phasen wurden getrennt und die wässrige Phase gründlich mit Dichlormethan extrahiert. Die vereinigten organischen Phasen wurden mit Natriumthiosulfatlösung und Wasser gewaschen, getrocknet und eingeengt. Destillation lieferte 450 g farblose Flüssigkeit; Sdp. = 65°C bei 15 mbar.

### Beispiel 9

### Herstellung von 2,4-Difluor-3-methylbenzoesäure

13 g (534 mmol) Magnesium wurden unter Stickstoff in 600 ml Tetrahydrofuran vorgelegt und über Nacht mit einem Magnetrührer langsam gerührt. Der Rührer wurde abgestellt, man gab zunächst einen Iod-Kristall zu, dann tropfte man ca. 10 g 3-Brom-2,6-difluortoluol aus Beispiel 8 zu, bis die Reaktion ansprang. Anschließend wurden unter Rühren die restlichen 90 g 3-Brom-2,6-difluortoluol (insgesamt 483 mmol) innerhalb von 65 Minuten zugetropft, so dass ein kontinuierlicher leichter Rückfluss erhalten wurde (Eigenwärme). Nach Ende der Zugabe wurde nachgerührt, bis eine klare Lösung erhalten wurde. Anschließend wurde ca. 35 Minuten lang Kohlendioxid eingeleitet, bis keine Exothermie mehr beobachtet werden konnte. Man tropfte vorsichtig 500 ml Wasser zu. Die restlichen Mg-Späne wurden abfiltriert und das Filtrat mit ca. 250 ml konzentrierter Salzsäure angesäuert (pH 1). Der dabei ausfallende Feststoff wurde abgesaugt und im Vakuum getrocknet. Umkristallisieren aus Ethanol lieferte 38 g farblosen Feststoff; Schmp.: 168 - 170°C.

### Beispiel 10

### Herstellung von 2,4-Difluor-3-methylbenzoesäurechlorid

25.0 g (145 mmol) 2,4-Difluor-3-methylbenzoesäure aus Beispiel 9 und 2.1 g (29 mmol) Dimethylformamid wurden unter Stickstoff in 300 ml Dichlormethan vorgelegt und 26 g (217 mmol) Thionylchlorid innerhalb von 5 Minuten zugetropft. Man erhitzte 70 Stunden auf Rückfluss und destillierte dann Thionylchlorid und Dichlormethan ab. Man erhielt 22 g eines farblosen Feststoffs.

### Beispiel 11

### Herstellung von 2,4-Difluor-3-methylbenzoesäurenitril

100 g (483 mmol) 3-Brom-2,6-difluortoluol, 52 g (580 mmol) Kupfercyanid und 9,2 g (48 mmol) Kupferiodid wurden unter Stickstoff in 700 ml Dimethylformamid vorgelegt. Man erhitzte 18 Stunden lang unter Rückfluss. Der entstandene Feststoff wurde abfiltriert und mehrfach mit wenig Methyl-tert.butylether gewaschen. Das Filtrat wurde mit 400 ml Wasser versetzt und die organischen Phase abgetrennt. Dabei bildete sich erneut ein Niederschlag, der abgesaugt wurde. Die wässrige Phase wurde viermal mit je ca. 200 ml Methyl-tert.butylether extrahiert. Die vereinigten organischen Phasen wurden mit Natriumchloridlösung gewaschen, mit Magnesiumsulfat getrocknet und eingeengt. Nach Destillation und Umkristallisieren aus Ethanol erhielt man 49 g farblose Kristalle; Schmp.: 40 - 44°C.

### Beispiel 12

### Herstellung von 2,4-Difluor-3-methylbenzaldehyd

Unter Stickstoff wurden 1.3 g (53 mmol) Magnesium in 20 ml Tetrahydrofuran vorgelegt und über Nacht mit einem Magnetrührer langsam gerührt. Der Rührer wurde abgestellt und ein Iod-Kristall zur Lösung gegeben. Dann tropfte man 10 g (48 mmol) 3-Brom-2,4-difluortoluol aus Beispiel 8 in 15 ml Tetrahydrofuran so langsam zu, dass ein kontinuierlicher leichter Rückfluss erhalten wurde (Eigenwärme). Nach beendeter der Zugabe wurde nachgerührt, bis eine klare Lösung entstand (ca. 2 Stunden). Dann tropft man innerhalb von 100 Minuten bei Raumtemperatur 7.1 g (97 mmol) Dimethylformamid in 35 ml Tetrahydrofuran zu. Die Reaktionsmischung wurde 4 Stunden unter Rückfluss erhitzt, dann durch Gießen auf Eiswasser hydrolysiert. Die restlichen Magnesium-Späne wurden abfiltriert. Man stellte die Suspension mit konzentrierte Salzsäure sauer (pH 1). Die wässrige Phase extrahierte man 3 mal mit jeweils 50 ml Ethylacetat. Die vereinigten organischen Phasen wurden mit 100 ml gesättigter Natriumchloridlösung gewaschen, getrocknet und eingeengt.

Das so erhaltene Rohprodukt enthielt 12 % an gewünschtem Zielprodukt und höhermolekulare Komponenten.

### Beispiel 13

### Herstellung von 2,4-Difluor-3-methylbenzylamin

8.8 g (58 mmol) 2,4-Difluor-3-methylbenzoesäurenitril aus Beispiel 11 wurden unter Stickstoff in 70 ml Tetrahydrofuran aufgenommen. Zu dieser Lösung wurden 4.4 g (116 mmol) Lithiumaluminiumhydrid portionsweise zugegeben und anschließend 10 Stunden bei Raumtemperatur nachgerührt. Man hydrolysierte überschüssiges Lithiumaluminiumhydrid durch vorsichtige Zugabe von 150 ml Wasser und filtrierte den entstandenen Niederschlag ab. Das erhaltene Filtrat wurde mit Wasser gewaschen und die vereinigten wässrigen Phasen mit Methyl-tert.butylether extrahiert. Die vereinigten organischen Phasen wurden mit gesättigter Natriumchloridlösung gewaschen, mit Magnesiumsulfat getrocknet und eingeengt.

Man erhielt 8.2 g einer farblosen Flüssigkeit, die laut GC-MS 76 % des Zielproduktes enthielt.

### Beispiel 14

### Herstellung von 2,4-Difluor-3-methylbenzylalkohol

11 g (58 mmol) 2,4-Difluor-3-methylbenzoesäurechlorid aus Beispiel 10 wurden unter Stickstoff in einem Gemisch aus 80 ml Dimethylformamid und 80 ml Tetrahydrofuran vorgelegt. Man gab portionsweise 4.4 g (115 mmol) Natriumborhydrid zu und ließ anschließend noch 18 Stunden bei Raumtemperatur rühren. Das Reaktionsgemisch wurde unter Eiskühlung mit 200 ml Eiswasser versetzt und dreimal mit jeweils 100 ml Dichlormethan extrahiert. Die vereinten organischen Phasen wurden über Magnesiumsulfat getrocknet und eingeengt.

Man erhielt 8.5 g einer farblosen Flüssigkeit, die laut GC-MS 80 % des Zielproduktes enthielt.

## Patentansprüche

1. Verbindungen der Formel (I) 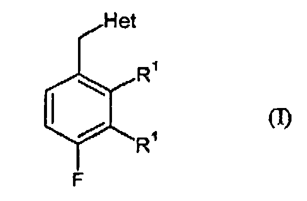 in der
Het für Amino oder Hydroxy steht und einer der Reste R¹ für Fluor und der andere Rest R¹ für C₁-C₄-Alkyl steht.

2. Verbindungen nach Anspruch 1, **dadurch gekennzeichnet, dass** die Verbindungen ausgewählt sind aus 3,4-Difluor-2-methyl-benzylalkohol, 2,4-bifluor-3-methyl-benzylalkohol, 3,4-Difluor-2-methyl-benzylamin und 2,4-Difluor-3-methyl-benzylamin.

3. Verfahren zur Herstellung von Verbindungen gemäß Formel (I) nach Anspruch 1 oder 2, **dadurch gekennzeichnet, dass**
• in einem Schritt A)
Verbindungen der Formel (II) 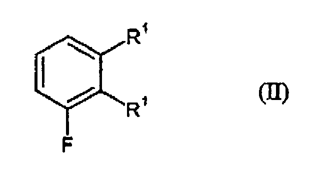 in Gegenwart von Brom und einem Katalysator zu Verbindungen der Formel (III) umgesetzt werden 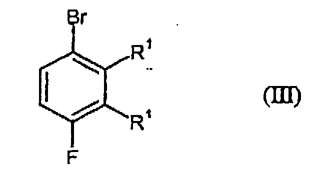 und für den Fall, dass Het für Amino steht, die Verbindungen der Formel (III)
• in einem Schritt B mit Cyanid zunächst zu Verbindungen der Formel (IV) 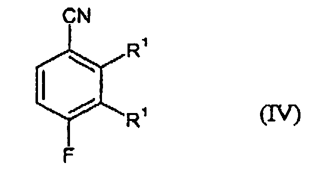 und anschließend
• in einem Schritt B2 durch Reduktion in die entsprechenden Verbindungen der Formel (I) überführt werden oder
für den Fall, dass Het für Hydroxy steht,
• in einem Schritt B3 die Verbindungen der Formel (III) durch Überführung in eine magnesiumorganische Verbindung, Umsetzung dieser magnesiumorganischen Verbindung mit Kohlendioxid und anschließender Behandlung mit Säure zu Verbindungen der Formel (V) umsetzt 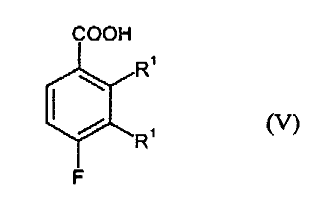 und die Verbindungen der Formel (V)
• in einem Schritt B4 durch Umsetzung mit einem Halogenierungsmittel in Verbindungen der Formel (VI) überführt werden 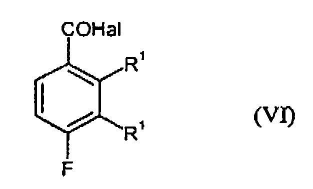 in der Hal für Brom oder Chlor steht
• und in einem Schritt B5 die Verbindungen der Formel (VI) durch Reduktion in die entsprechenden Verbindungen der Formel (I) überführt werden.

4. Verfahren nach Anspruch 3, **dadurch gekennzeichnet, dass** alternativ zu den Schritten B3 bis B5
• in einem Schritt B6 die Umsetzung der Verbindungen der Formel (III) durch Überführung in eine magnesiumorganische Verbindung, Umsetzung dieser magnesiumorganischen Verbindung mit einem N,N-substituierten-Ameisensäureamid und anschließender Behandlung mit Säure zu Verbindungen der Formel (VII) erfolgt 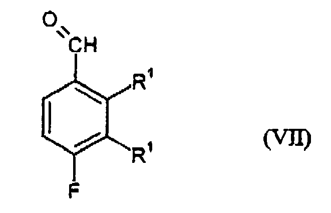 wobei dann
• in einem Schritt B7 die Verbindungen der Formel (VII) durch Reduktion in die entsprechenden Verbindungen der Formel (I) überführt werden.

5. Verbindungen der Formel (III) gemäß Anspruch 3 mit Ausnahme von 3-Brom-2,6-difluortoluol,

6. Verbindungen der Formel (IV) gemäß Anspruch 3.

7. Verbindungen der Formel (V) gemäß Anspruch mit Ausnahme von 2,4-Difluor-3-methylbenzoesäure und 3,4-Difluor-2-methylbenzoesäure.

8. Verbindungen der Formel (VI) gemäß Anspruch 3 mit Ausnahme von 2,4-Difluor-3-methylbenzoesäure-chlorid

9. Verwendung von Verbindungen gemäß Ansprüchen 1, 2 und 5 bis 8 oder solchen Verbindungen, die nach zumindest einem der Ansprüche 3 und 4 hergestellt wurden in einem Verfahren zur Herstellung von Wirkstoffen in Arzneimitteln und Pflanzenschutzmitteln oder Zwischenprodukten davon.

## Claims

1. Compounds of the formula (I) 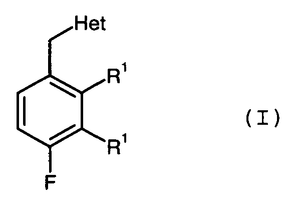 in which
Het is amino or hydroxyl and one of the R¹ radicals is fluorine and the other R¹ radical is C₁-C₄-alkyl .

2. Compounds according to Claim 1, **characterized in that** the compounds are selected from 3,4-difluoro-2-methylbenzyl alcohol, 2,4-difluoro-3-methylbenzyl alcohol, 3,4-difluoro-2-methylbenzylamine and 2,4-difluoro-3-methylbenzylamine.

3. Process for preparing compounds of the formula (I) according to Claim 1 or 2, **characterized in that**,
• in a step A)
compounds of the formula (II) 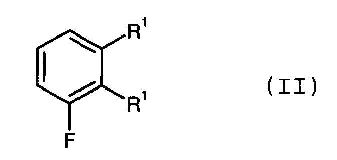 are converted in the presence of bromine and a catalyst to compounds of the formula (III) 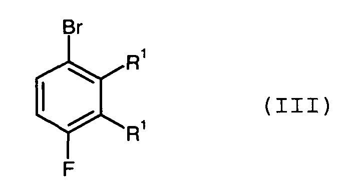 and, in the case that Het is amino, the compounds of the formula (III),
• in a step B1, are reacted with cyanide initially to give compounds of the formula (IV) 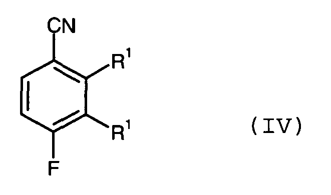 and subsequently,
• in a step B2, converted by reduction to the corresponding compounds of the formula (I), or,
in the case that Het is hydroxyl,
• in a step B3, the compounds of the formula (III) are converted by conversion to an organomagnesium compound, reaction of this organomagnesium compound with carbon dioxide and subsequent treatment with acid to give compounds of the formula (V) 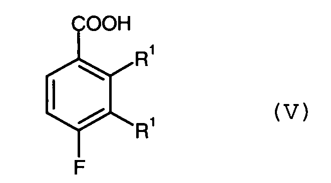 and the compounds of the formula (V),
• in a step B4, are converted using a halogenating agent to compounds of the formula (VI) 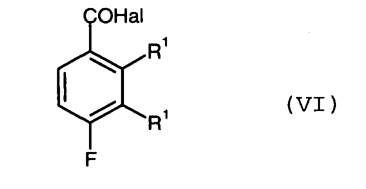 in which Hal is chlorine or bromine
• and, in a step B5, the compounds of the formula (VI) are converted by reduction to the corresponding compounds of the formula (I).

4. Process according to Claim 3, **characterized in that**, alternatively to the steps B3 to B5,
• in a step B6, the compounds of the formula (III) are converted to an organomagnesium compound, this organomagnesium compound is reacted with an N,N-substituted formamide and subsequently treated with acid to give compounds of the formula (VII) 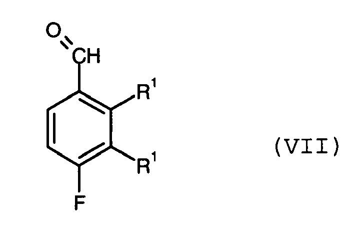 and then,
• in a step B7, the compounds of the formula (VII) are converted by reduction to the corresponding compounds of the formula (I).

5. Compounds of the formula (III) according to claim 3 with the exception of 3-bromo-2,6-difluorotoluene.

6. Compounds of the formula (IV) according to Claim 3.

7. Compounds of the formula (V) according to Claim 3 with the exception of 2,4-difluoro-3-methylbenzoic acid and 3,4-difluoro-2-methylbenzoic acid.

8. Compounds of the formula (VI) according to Claim 3 with the exception of 2,4-difluoro-3-methylbenzoyl chloride.

9. Use of compounds according to Claims 1, 2 and 5 to 8 or those compounds which have been prepared according to at least one of Claims 3 and 4 in a process for preparing active ingredients in pharmaceuticals and crop protection agents or intermediates thereof.

## Revendications

1. Composés de formule (I) 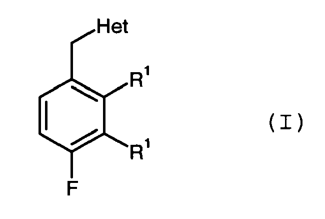 dans laquelle
Het représente un groupe amino ou un groupe hydroxy et l'un des radicaux R¹ représente un atome de fluor et l'autre radical R¹ représente un groupe alkyle en C₁-C₄.

2. Composés selon la revendication 1, **caractérisés en ce que** les composés sont choisis parmi l'alcool 3,4-difluoro-2-méthylbenzylique, l'alcool 2,4-difluoro-3-méthylbenzylique, la 3,4-difluoro-2-méthylbenzylamine et la 2,4-difluoro-3-méthylbenzylamine.

3. Procédé de préparation de composés de formule (I) selon la revendication 1 ou 2, **caractérisé en ce que**
• dans une étape A)
des composés de formule (II) 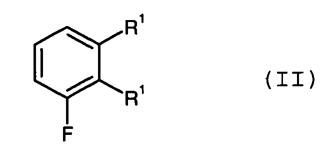 sont mis à réagir en présence de brome et d'un catalyseur pour donner lieu à des composés de formule (III) 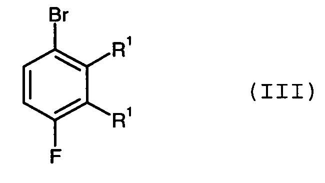 et, au cas où Het représente un groupe amino, les composés de formule (III),
• dans une étape B1, sont d'abord transformés avec du cyanure en des composés de formule (IV) 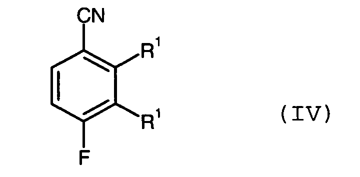 et ensuite
• dans une étape B2, par réduction en les composés correspondants de formule (I), ou,
au cas où Het représente un groupe hydroxy,
• dans une étape B3, les composés de formule (III) sont mis à réagir par transformation en un composé organo-magnésium, par réaction de ce composé organo-magnésium avec du dioxyde de carbone et par traitement subséquent avec un acide, pour donner lieu à des composés de formule (V) 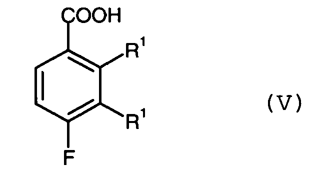 et les composés de formule (V),
• dans une étape B4, sont transformés par réaction avec un agent d'halogénation en des composés de formule (VI) 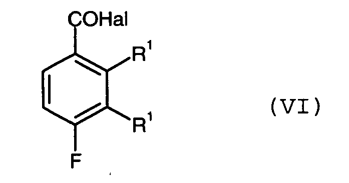 dans laquelle Hal représente un atome de brome ou un atome de chlore,
• et, dans une étape B5, les composés de formule (VI) sont transformés par réduction en les composés correspondants de formule (I) .

4. Procédé selon la revendication 3, **caractérisé en ce que**, en variante aux étapes B3 à B5,
• dans une étape B6, la réaction des composés de formule (III) est réalisée par transformation en un composé organo-magnésium, par réaction de ce composé organo-magnésium avec un formamide N,N-substitué et par traitement subséquent avec un acide, pour donner lieu à des composés de formule (VII) 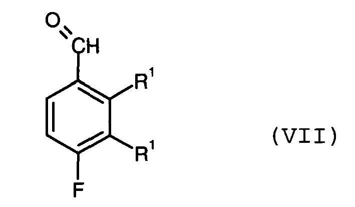 où,
• dans une étape B7, les composés de formule (VII) sont alors transformés par réduction en les composés correspondants de formule (I).

5. Composés de formule (III) selon la revendication 3, à l'exception du 3-bromo-2,6-difluorotoluène.

6. Composés de formule (IV) selon la revendication 3.

7. Composés de formule (V) selon la revendication 3, à l'exception de l'acide 2,4-difluoro-3-méthylbenzoïque et de l'acide 3,4-difluoro-2-méthylbenzoïque.

8. Composés de formule (VI) selon la revendication 3, à l'exception du chlorure d'acide 2,4-difluoro-3-méthylbenzoïque.

9. Utilisation de composés selon les revendications 1, 2 et 5 à 8 ou des composés qui ont été préparés selon au moins l'une des revendications 3 et 4 dans un procédé de préparation d'ingrédients actifs dans des médicaments et des agents phytoprotecteurs ou leurs intermédiaires.
